(19) Europäisches Patentamt
European Patent Office
Office européen des brevets

(11) **EP 3 411 007 B1**

(12) **EUROPÄISCHE PATENTSCHRIFT**

(45) Veröffentlichungstag und Bekanntmachung des Hinweises auf die Patenterteilung:
**05.08.2020 Patentblatt 2020/32**

(21) Anmeldenummer: **16822963.1**

(22) Anmeldetag: **19.12.2016**

(51) Int Cl.:
*A61K 8/06* (2006.01)   *A61K 8/58* (2006.01)
*A61K 8/92* (2006.01)   *A61Q 19/08* (2006.01)
*A61Q 17/04* (2006.01)   *A61Q 1/02* (2006.01)

(86) Internationale Anmeldenummer:
**PCT/EP2016/081685**

(87) Internationale Veröffentlichungsnummer:
**WO 2017/133822 (10.08.2017 Gazette 2017/32)**

(54) **WASSER-IN-ÖL-EMULSIONEN AUF DER GRUNDLAGE VON CETYL DIGLYCERYL TRIS(TRIMETHYLSILOXY)SILYLETHYLDIMETHICONE ALS EMULGATOR, WELCHE WEITGEHEND FREI SIND VON SILIKONÖLEN**

WATER-IN-OIL EMULSIONS BASED ON CETYL DIGLYCERYL TRIS (TRIMETHYLSILOXY) SILYLETHYLDIMETHICONE AS AN EMULGATOR, LARGELY FREE OF SILICONE OILS

ÉMULSIONS D'EAU DANS L'HUILE BASÉES SUR CETYL DIGLYCÉRYL TRIS (TRIMÉTHYLSILOXY) SILYL ETHYL DIMÉTHICONE COMME EMULGATEUR, EN GRANDE PARTIE LIBRE DE L'HUILE DE SILICONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.02.2016 DE 102016201763**

(43) Veröffentlichungstag der Anmeldung:
**12.12.2018 Patentblatt 2018/50**

(73) Patentinhaber: **Beiersdorf AG**
**20253 Hamburg (DE)**

(72) Erfinder:
• **CHANTY, Delphine**
**22767 Hamburg (DE)**
• **RASCHKE, Thomas**
**25421 Pinneberg (DE)**

(56) Entgegenhaltungen:
**EP-A2- 1 022 055    DE-U1- 20 221 822**

• **Cindy Delvallé ET AL: "New Formulation Possibilities with a Water-in-oil Silicone Emulsifier Suitable for PEG-free Systems", , 31. Dezember 2014 (2014-12-31), XP055349098, Gefunden im Internet: URL:http://www.dowcorning.com/content/publishedlit/27-1463-water-in-oil-silicone-emulsifier.pdf [gefunden am 2017-02-23]**

EP 3 411 007 B1

**Beschreibung**

Die vorliegende Erfindung betrifft eine kosmetische Zubereitung Zubereitungen in Form von Wasser-in-Öl-Emulsionen auf der Grundlage von Cetyl Diglyceryl Tris-(Trimethylsiloxy)silyl-ethyldimethicone als Emulgator, und welche weitgehend frei sind von Silikonölen.

Anwender stellen an moderne Hautpflegeprodukte, speziell in Form von Cremes für die Anwendung im Gesicht, eine Reihe von Anforderungen. Sie sollen die Haut pflegen, insbesondere die Haut über einen langen Zeitraum mit Feuchtigkeit versorgen. Dieses wird in der Regel über "Moisturizer" realisiert, die Wasser im Stratum Corneum binden (z.B. Glycerin), zudem werden mit zugesetzten Lipiden die oberen Hautschuppen geschmeidig gehalten und der transepidermale Wasserverlust reduziert.

Des Weiteren erwarten die Anwender häufig eine Wirkung gegen die Anzeichen von Hautalterung, wie z.B. die Ausbildung von Falten, das Nachlassen der Hautelastizität oder die Entstehung von Altersflecken. Hierzu werden zum einen Wirkstoffe verwendet, die beispielsweise die Aktivität der Hautzellen stimulieren, aber auch verstärkt UV-Filter eingesetzt, die besonders für die Hautalterung verantwortlichen UVA-Strahlen blockieren. Alle diese Inhaltsstoffe stellen eine große Herausforderung für eine angenehme Sensorik dar, da sie nach dem Verdunsten der flüchtigen Bestandteile der Formulierung als Film auf der Haut zurückbleiben, und dort sensorisch vom Verbraucher wahrgenommen werden. Die fühlbaren Rückstände werden dabei oft negativ beschrieben und verhindern das Gefühl, daß das Produkt komplett in die Haut "eingezogen" sei.

Konventionelle W/O Emulsionen werden gerne von Verbrauchern verwendet, wenn sie eine trockene Haut pflegen oder behandeln wollen. Vorteile bieten W/O Emulsionen auch bei der Einarbeitung von Farbpigmenten, z.B. in getönten Tagescremes und Make-up. Leider zeichnen sich viele W/O Emulsionen aber dadurch negativ aus, dass sie einen öligen Rückstand auf der Haut hinterlassen, der nur langsam einzieht. Darüber hinaus ist ihre physikalische Stabilität oft begrenzt. Das ist besonders dann problematisch, wenn W/O Emulsionen mit polaren Ölen hergestellt werden sollen. Sensorisch hochwertige W/O Emulsionen lassen sich mit Emulgatoren auf Basis von Silicon-Derivaten herstellen. Allerdings enthalten diese Rezepturen meist sehr hohe Mengen an Siliconölen, da diese sich besonders gut mit Siliconemulgatoren emulgieren lassen. Leider weisen Siliconöle aber einige erhebliche Nachteile auf. Ihre Lösungseigenschaften für kosmetische Rohstoffe sind sehr gering und die sensorischen Eigenschaften sind limitiert. So lassen sich beispielsweise UV-Filter nur sehr begrenzt in Siliconölen bzw Siliconemulsionen lösen.

Ein weiteres Problem des Standes der Technik ist, daß es meist anspruchsvoller Entwicklungsarbeit bedarf, um niedrigviskose W/O-Emulsionen, zumal solche mit erhöhtem Wassergehalt zu formulieren.

Unter dem Begriff "Viskosität" versteht man die Eigenschaft einer Flüssigkeit, der gegenseitigen laminaren Verschiebung zweier benachbarter Schichten einen Widerstand (Zähigkeit, innere Reibung) entgegenzusetzen. Man definiert heute diese sogenannte dynamische Viskosität nach $\eta = \tau/D$ als das Verhältnis der Schubspannung zum Geschwindigkeitsgradienten senkrecht zur Strömungsrichtung. Für newtonsche Flüssigkeiten ist $\eta$ bei gegebener Temperatur eine Stoffkonstante mit der SI-Einheit Pascalsekunde (Pa·s).

Der Quotient $v = \eta/\rho$ aus der dynamischen Viskosität $\eta$ und der Dichte $\rho$ ("rho") der Flüssigkeit wird als kinematische Viskosität $v$ ("ny") bezeichnet und in der SI-Einheit $m^2/s$ angegeben.

Als Fluidität ($\varphi$) bezeichnet man den Kehrwert der Viskosität ($\varphi = 1/\eta$). Bei Salben und dergleichen wird der Gebrauchswert unter anderem mitbestimmt von der sogenannten Zügigkeit. Unter der Zügigkeit einer Salbe oder Salbengrundlage oder dergleichen versteht man deren Eigenschaft, beim Abstechen verschieden lange Fäden zu ziehen; dementsprechend unterscheidet man kurz- und langzügige Stoffe.

Während die graphische Darstellung des Fließverhaltens newtonscher Flüssigkeiten bei gegebener Temperatur eine Gerade ergibt, zeigen sich bei den sogenannten nichtnewtonschen Flüssigkeiten in Abhängigkeit vom jeweiligen Geschwindigkeitsgefälle D (Schergeschwindigkeit $\dot{\gamma}$) bzw. der Schubspannung $\tau$ oft erhebliche Abweichungen. In diesen Fällen läßt sich die sogenannte scheinbare Viskosität bestimmen, die zwar nicht der Newtonschen Gleichung gehorcht, aus der sich jedoch durch graphische Verfahren die wahren Viskositätswerte ermitteln lassen.

Die Fallkörperviskosimetrie ist lediglich zur Untersuchung newtonscher Flüssigkeiten sowie von Gasen geeignet. Sie basiert auf dem Stokes-Gesetz, nach dem für das Fallen einer Kugel durch eine sie umströmende Flüssigkeit die dynamische Viskosität $\eta$ aus

$$\eta = \frac{2\,r^2(\rho_K - \rho_{Fl})\cdot g}{9\cdot v}$$

bestimmbar ist, wobei

r = Radius der Kugel,
V = Fallgeschwindigkeit,

$\rho_K$ = Dichte der Kugel,
$\rho_{Fl}$ = Dichte der Flüssigkeit und
g = Fallbeschleunigung.

Niedrige Viskositäten im Sinne der vorliegenden Erfindung liegen im Intervall von 100 - 20000 mPa.s, vorzugsweise von 200 - 10000 mPa.s, insbesondere bevorzugt von 300 - 5000 mPa.s bei einer Temperatur von 25 °C und einer Scherrate von ca. 10 s$^{-1}$ liegen.

Silikonbasierte Emulgatoren haben fast ausnahmslos PEG- und PPG-haltige Gruppen als hydrophile Komponente. Typische Vertreter sind Cetyl PEG/PPG-10/1 Dimethicone (Abil EM 90) von Evonik oder PEG-10 Dimethicone (ES-5612) von Dow Corning. Diese Beispiele sind erfolgreiche und seit langem etablierte W/Si Emulgatoren im Markt. Die Auswahl an PEGfreien Silikonemulgatoren ist aber beschränkt, man findet hier beispielsweise KF-6100, KF-6104, KF-6105 oder KSG-710, KSG-810, KSG-820, KSG-830 oder KSG-840 von Shin Etsu oder Abil EM 120 von Evonik.

Üblicherweise werden diese W/Si Silikonemulgatoren in hohen Konzentrationen von mehreren Prozent eingesetzt. Die Auswahl der Öle für W/O Emulsionen sowie insbesondere für W/Si Emulsionen ist begrenzt, da für eine gute Langzeitstabilität ein hoher Anteil an unpolaren Ölen erforderlich ist. Daher kommen in W/Si Emulsionen im Wesentlichen Silikonöle und/oder flüssige Kohlenwasserstoffe zum Einsatz.

Aufgabe der vorliegenden Erfindung ist daher, eine stabile (Lagerstabile, transportstabile, langzeitstabile) Wasser-in-Silikonöl- (W/Si-) Emulsion zur Verfügung zu stellen, die sensorisch attraktiv, PEG-frei und weitgehend frei von Siliconölen ist, dabei aber alle Anforderungen an ein modernes Gesichtspflege-Produkt erfüllt.

Insgesamt bleibt das Hauptproblem, daß Wasser-in-Silikonöl-Emulsionen zu Instabilität neigen. Bekannte Übelstände dieser Art sind Ölabscheidung und Phasentrennung. Das Problem wird noch größer, wenn mineralische Pigmente eingebaut werden müssen, wie es beispielswiese in sogenannten "Foundations" der Fall ist. Zwar kann dem durch Verwendung bekannter Stabilisatoren (beispielsweise Hektorite) entgegengewirkt werden, oder der Anteil der Ölphase kann vermindert werden. Dadurch müssen dann aber wiederum andere Nachteile in Kauf genommen werden, beispielsweise, daß sich polare lipophile Substanzen wie beispielsweise UV-Filter schlechter einarbeiten lassen.

Aufgabe der vorliegenden Erfindung war es daher, diesen Übelständen entgegenzuwirken. Ein besonders interessanter Emulgator wird von Dow Corning unter der INCI- Bezeichnung Cetyl Diglyceryl Tris-(Trimethylsiloxy)silylethyldimethicone und unter der Handelsbezeichung ES-5600 verkauft. In "Cindy Delvallé ET AL: "New Formulation Possibilities with a Water-in-oil Silicone Emulsifier Suitable for PEG-free Systems", 31. Dezember 2014 (2014-12-31), Dow Corning" wird konkret das Cetyl/Diglyceryl/Tris(Trimethylsiloxy)silylethyl-Dimethicon (ES5600) als hinsichtlich Silikonöl-Anteil flexibler Emulgator für W/O Emulsionen beschrieben.

Silikonemulgatoren allerdings werden klassischerweise verwendet, um Wasser-in-Silikonöl-Emulsionen oder Silikonöl-in- Wasser-Emulsionen zu erzeigen. Als Emulgatoren für Wasser-in-ÖI-Emulsionen oder Öl-in-Wasser-Emulsionen unter Verwendung von nicht-Siliconölen sind sie normalerweise nicht geeignet.

Es war daher überraschend und für den Fachmann nicht vorhersehbar, daß die der Erfindung zugrundeliegenden Aufgaben gelöst werden durch kosmetische oder dermatologische Wasser-in-ÖI Emulsionen, umfassend

A) Cetyl Diglyceryl Tris-(Trimethylsiloxy)silylethyldimethicone als Emulgator
B) eine polare Ölphase mit maximal 0,95 Gewichts-%, bevorzugt höchstens 0,5 Gewichts-%, besonders bevorzugt höchstens 0,1 Gewichts-% an einem oder mehreren bei Raumtemperatur flüssigen Silikonölen enthalten und ganz besonders bevorzugt gänzlich frei von Silikonölen sind,
C) eine wässrige Phase.

Erfindungsgemäß enthalten Wasser-in-Öl-Emulsionen 0,1 bis 10,0 Gew.-%, bevorzugt 0,2 bis 5,0 Gew.-%, besonders bevorzugt 0,5 bis 4,0 Gew.-%, bezogen auf das Gesamtgewicht der Zubereitung, an einem oder mehreren erfindungsgemäßen Silikonemulgatoren.

Es war nicht zu erwarten gewesen, dass die erfindungsgemäßen weitgehend oder vollständig silikonölfreien Zubereitungen

- durch Silikonemulgatoren stabilisiert werden
- besser als feuchtigkeitsspendende Zubereitungen wirken,
- einfacher zu formulieren sein,
- besser die Hautglättung fördern,
- sich durch besser Pflegewirkung auszeichnen,
- besser als Vehikel für kosmetische und dermatologische Wirkstoffe dienen
- bessere sensorische Eigenschaften, wie beispielsweise die Verteilbarkeit auf der Haut oder das Einzugsvermögen in die Haut, aufweisen würden
- besser als Vehikel für organische UV-Filter geeignet sind

- höhere Stabilität gegenüber Zerfall in Öl- und Wasserphasen aufweisen und
- sich durch bessere Bioverträglichkeit auszeichnen würden
- die Herstellung von stabilen, insbesondere dünnflüssigen Formulierungen ermöglichen

als die Zubereitungen des Standes der Technik.

Die erfindungsgemäßen Zubereitungen sind fließfähig aber auch cremeartig formulierbar, besitzen sehr gute kosmetische Eigenschaften, insbesondere was die Klebrigkeit betrifft, und weisen sehr gute Hautverträglichkeit sowie Hautpflegeleistung auf.

Erfindungsgemäß ist es möglich und vorteilhaft, den Anteil der Ölphase der erfindungsgemäßen Zubereitungen im Bereich von 3 bis 60 Gew.-%, bezogen auf das Gesamtgewicht der Zubereitungen, frei zu wählen.

Die erfindungsgemäße Öl- bzw. Lipidphase kann dabei alle in kosmetischen Zubereitungen üblichen Öle, Fette, Wachse und/oder Lipide enthalten.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte, Phenoxyethanol, Methylparaben, Schaumstabilisatoren, Elektrolyte, Selbstbräuner etc.

Als Grundbestandteile der erfindungsgemäßen Zubereitungen können verwendet werden:

- Wasser oder wässrige Lösungen
- wässrige ethanolische Lösungen
- natürliche Öle und/oder chemisch modifizierte natürliche Öle und/oder synthetische Öle;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet.

Die Ölphase der Emulsionen im Sinne der vorliegenden Erfindung ist erfindungsgemäß im Wesentlichen frei von Silikonölen. Toleriert werden kann ein Gehalt von maximal 0,95 Gewichts-%, bevorzugt höchstens 0,5 Gewichts-%, noch besser höchstens 0,1 Gewichts-% an einem oder mehreren bei Raumtemperatur flüssigen Silikonölen.

Indessen ist es bevorzugt, die Ölkomponenten der Ölphase der erfindungsgemäßen Zubereitungen vollständig oder weitestgehend vollständig aus der Gruppe der Öle synthetischen und/oder natürlichen Ursprungs zu wählen. Diese können dann vorteilhaft gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, n-Decyloleat, Isooctylstearat, Isononylstearat, Isononylisononanoat, 2-Ethylhexylpalmitat, 2-Ethylhexyllaurat, 2-Hexyldecylstearat, Coco-Caprylat/Caprat, Oleyloleat, Oleylerucat, Erucyloleat, Erucylerucat sowie synthetische, halbsynthetische und natürliche Gemische solcher Ester, z.B. Jojobaöl.

Ferner kann die Ölphase teilweise vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Caprylic Capric Triglycerid, Sonnenblumenöl, Sojaöl, Mandelöl und dergleichen mehr.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Reiskeimölwachs, Beerenwachs, Montanwachs, Jojobawachs, Shea Butter, Bienenwachs, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifizierte Wachse und synthetische Wachse, wie beispielsweise die unter den Handelsbezeichnungen Syncrowax HRC (Glyceryltribehenat), Syncrowax

HGLC ($C_{16-36}$-Fettsäuretriglycerid) und Syncrowax AW 1C ($C_{18-36}$-Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder $C_{30-50}$-Alkyl Bienenwachs), Polyalkylenwachse, Polyethylenglykolwachse, aber auch chemisch modifizierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise $C_{20-40}$-Alkylstearat oder $C_{20-40}$-Alkylhydroxystearoylstearat.

Erfindungsgemäß können die Fett- und/oder Wachskomponenten sowohl einzeln als auch im Gemisch vorliegen.

Vorteilhaft werden die Ölkomponenten gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether sowie insbesondere Cetarylisononanoat.

Von den Kohlenwasserstoffen sind Paraffinöl, Squalan, hydriertes Polyisobuten, Isohexadecan, Isododecan; $C_{15-19}$Alkan bzw. Polydecen vorteilhaft im Sinne der vorliegenden Erfindung zu verwenden.

Zubereitungen entsprechend der vorliegenden Erfindung können auch einen oder mehrere zusätzliche Nicht-Silikon-Emulgatoren enthalten, beispielsweise vorteilhaft gewählt aus der Gruppe der folgenden Substanzen, die in der Regel als W/O-Emulgatoren wirken.

Sorbitanstearat, Sorbitanoleat, Glycerylisostearat, Polyglyceryl-3-Oleat, Wollwachssäuregemische, Wollwachsalkoholgemische, Polyglyceryl-3 Diisostearat, Methylglucosedioleat, Polyglyceryl-4-isostearat Cetyldimethiconcopolyol, Sorbitanisostearat, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Polyglyceryl-2-dipolyhydroxystearat, Polyglyceryl-3-dipolyhydroxystearat, Polyglyceryl-4-dipolyhydroxystearat.

Es war insbesondere überraschend, daß erfindungsgemäße kosmetische oder dermatologische Zubereitungen ganz besonders vorteilhafte Eigenschaften aufweisen. Insbesondere ist es erfindungsgemäß möglich, niedrigviskose Zubereitungen zu erstellen, deren Viskositäten im Intervall von 100 - 20000 mPa.s, vorzugsweise von 200 - 10000 mPa.s, insbesondere bevorzugt von 300 - 5000 mPa.s bei einer Temperatur von 25 °C und einer Scherrate von ca. 10 $s^{-1}$ liegen (gemessen mit dem Rheomat R 123).

Die Gesamtmenge an erfindungsgemäß verwendeten Silikonemulgatoren in den erfindungsgemäßen kosmetischen oder dermatologischen Zubereitungen wird vorteilhaft aus dem Bereich von 0,1 - 10,0 Gew.-%, bevorzugt 0,2 - 5,0 Gew.-% gewählt, bezogen auf das Gesamtgewicht der Zubereitungen.

Die Gesamtmenge an erfindungsgemäß verwendeten Nicht-Silikon-Emulgatoren in den erfindungsgemäßen kosmetischen oder dermatologischen Zubereitungen wird vorteilhaft aus dem Bereich von 0,001 - 10,0 Gew.-%, bevorzugt 0,01 - 5,0 Gew.-% gewählt, bezogen auf das Gesamtgewicht der Zubereitungen.

Erfindungsgemäße Emulsionen im Sinne der vorliegenden Erfindung, z.B. in Form einer Hautschutzcreme, einer Hautlotion, einer kosmetischen Milch, beispielsweise in Form einer Sonnenschutzcreme oder einer Sonnenschutzmilch, sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, Pigments, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelte entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Es ist ebenfalls von Vorteil, von den erfindungsgemäßen Eigenschaften in Form von dekorativen Kosmetika (Make-Up-Formulierungen) Gebrauch zu machen.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment. Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:

- Ester der Zimtsäure, vorzugsweise 4-Methoxyzimtsäure(2-ethylhexyl)ester
- Ester der Salicylsäure, vorzugsweise Salicylsäure(2-ethylhexyl)ester, Salicylsäure(4-isopropylbenzyl)ester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- 2,4,6-Trianilino-(p-carbo-2'-ethyl-1'-hexyloxy)-1,3,5-triazin

Als wasserlösliche Substanzen sind vorteilhaft:

- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Chroms, Cers und Mischungen davon, sowie Abwandlungen, bei denen die Oxide die aktiven Agentien sind. Aber auch Ultramarinblau und Perlglanzpigmente. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, organische Lösungsmittel oder Silikonderivate.

Die nachfolgenden Beispiele sollen die vorliegende Erfindung verdeutlichen.

Zur Herstellung der erfindungsgemäßen Emulsionen werden die lipophile Phase und die wässrige Phase separat eingewogen, wobei der W/Si-Emulgator zur lipophilen Phase (Ölphase) zugegeben wird. Die Wasserphase wird unter Rühren bei Raumtemperatur langsam zur Ölphase hinzugegeben und anschließend mit einem hochtourigen Mixer (z.B. einem ESGE-Stab) intensiv vermischt (ca. 2 Minuten).

Zur Bestimmung der Lagerstabilität werden alle Emulsionsproben in verschraubten 30 ml Gläsern bei unterschiedlichen Temperaturen gelagert: bei Raumtemperatur (20°C), bei Wechseltemperatur: -10/40 °C; +40 °C.

Die Stabilität wurde nach 7 Tagen (-10/40°C), 1 Monat und 3 Monaten visuell bewertet.

Beispiele mit Emulgator Cetyl Diglyceryl Tris(Trimethylsiloxy)silylethyldimethicone (ES-5600):

| Beispielrezepturen | Menge Emulgator | -10/40 °C | RT | 40 °C |
|---|---|---|---|---|
| Bsp. 1 16% Cyclomethicone | a. 1% | Stabil | Leichte In-stabilität | Stabil |
| | b. 3% | Stabil | Stabil | Stabil |
| Bsp. 2 16% Cetaryl isononanoate | a. 1% | Stabil | Stabil | Stabil |
| | b. 3% | Stabil | Stabil | Stabil |
| Bsp. 3 16% Isohexadecane | a. 1 % | Stabil | Stabil | Stabil |
| | b. 3% | Stabil | Stabil | Stabil |

Beispiel mit Emulgator PEG-10 Dimethicone (ES-5612):

| Beispielrezepturen | Menge Emulgator | -10/40 °C | RT | 40 °C |
|---|---|---|---|---|
| Bsp. 4 16% Dimethicone | 1 % | Leichte In-stabilität | Leichte In-stabilität | Leichte In-stabilität |
| Bsp. 5 16% Cetaryl isononanoate | 1 % | Instabil | Instabil | Instabil |
| Bsp. 6 16% Isohexadecane | 1 % | Stabil | Stabil | Stabil |

Vergleich mit Emulgator Cetyl PEG/PPG-10/1 Dimethicone (Abil EM 90):

| Beispielrezepturen mit Pigmenten und 3% Emulgator | Emulgator | -10/40 °C | RT | 40 °C |
|---|---|---|---|---|
| Bsp. 7 16% Cetaryl isononanoate | Abil EM 90 | Instabil | Instabil | Instabil |
| Bsp. 8 16% Cetaryl isononanoate | ES-5600 | Stabil | Stabil | Stabil |

"Stabil" bedeutet, daß keine oder nur geringe Phasentrennung, keine oder nur geringe Ölabscheidung, keine visuell erkennbaren Inhomogenität der Pigmentverteilung auftritt. "Leichte Instabilität bedeutet, daß es zu einer moderaten, sichtbaren Phasentrennung oder deutlich sichtbaren Ölabscheidung oder zu visuell erkennbaren Inhomogenitäten kommt. "Instabil" bedeutet schnelle, vollständige Phasentrennung.

Die Verwendung von ES-5600 hat somit einen deutlichen Vorteil gegenüber bekannten W/Si-Emulgatoren bei der stabilen Emulgierung von polaren Ölphasen ohne Siliconöle sowie bei der Dispergierung von Pigmenten.

| Beispielrezeptur 1a | Gew.-% |
|---|---|
| Cetyl Diglyceryl Tris(Trimethylsiloxy)silylethyldimethicone | 1 |
| Cyclomethicon | 16 |
| Phenoxyethanol | 0,6 |
| EDTA | 1 |
| Glycerin | 6 |
| Natriumchlorid | 2 |
| Wasser 100% | ad 100 |

| Beispielrezeptur 1b | Gew.-% |
|---|---|
| Cetyl Diglyceryl Tris(Trimethylsiloxy)silylethyldimethicone | 3 |
| Cyclomethicon | 16 |
| Phenoxyethanol | 0,6 |
| EDTA | 1 |
| Glycerin | 6 |
| Natriumchlorid | 2 |
| Wasser 100% | ad 100 |

| Beispielrezeptur 2a | Gew.-% |
|---|---|
| Cetyl Diglyceryl Tris(Trimethylsiloxy)silylethyldimethicone | 1 |
| Cetaryl isononanoate | 16 |
| Phenoxyethanol | 0,6 |
| EDTA | 1 |
| Glycerin | 6 |
| Natriumchlorid | 2 |
| Wasser 100% | ad 100 |

| Beispielrezeptur 2b | Gew.-% |
|---|---|
| Cetyl Diglyceryl Tris(Trimethylsiloxy)silylethyldimethicone | 3 |
| Cetaryl isononanoate | 16 |
| Phenoxyethanol | 0,6 |
| EDTA | 1 |
| Glycerin | 6 |
| Natriumchlorid | 2 |

(fortgesetzt)

| Beispielrezeptur 2b | Gew.-% |
|---|---|
| Wasser 100% | ad 100 |

| Beispielrezeptur 3a | Gew.-% |
|---|---|
| Cetyl Diglyceryl Tris(Trimethylsiloxy)silylethyldimethicone | 1 |
| Isohexadecane | 16 |
| Phenoxyethanol | 0,6 |
| EDTA | 1 |
| Glycerin | 6 |
| Natriumchlorid | 2 |
| Wasser 100% | ad 100 |

| Beispielrezeptur 3b | Gew.-% |
|---|---|
| Cetyl Diglyceryl Tris(Trimethylsiloxy)silylethyldimethicone | 3 |
| Isohexadecane | 16 |
| Phenoxyethanol | 0,6 |
| EDTA | 1 |
| Glycerin | 6 |
| Natriumchlorid | 2 |
| Wasser 100% | ad 100 |

| Beispielrezeptur 4 | Gew.-% |
|---|---|
| PEG-10 Dimethicone | 1 |
| Dimethicone | 16 |
| Phenoxyethanol | 0,6 |
| EDTA | 1 |
| Glycerin | 6 |
| Natriumchlorid | 2 |
| Wasser 100% | ad 100 |

| Beispielrezeptur 5 | Gew.-% |
|---|---|
| PEG-10 Dimethicone | 1 |
| Cetaryl isononanoate | 16 |
| Phenoxyethanol | 0,6 |
| EDTA | 1 |
| Glycerin | 6 |

(fortgesetzt)

| Beispielrezeptur 5 | Gew.-% |
|---|---|
| Natriumchlorid | 2 |
| Wasser 100% | ad 100 |

| Beispielrezeptur 6 | Gew.-% |
|---|---|
| PEG-10 Dimethicone | 1 |
| Isohexadecane | 16 |
| Phenoxyethanol | 0,6 |
| EDTA | 1 |
| Glycerin | 6 |
| Natriumchlorid | 2 |
| Wasser 100% | ad 100 |

| Beispielrezeptur 7 | Gew.-% |
|---|---|
| Cetyl PEG/PPG-10/1 Dimethicone (Abil EM 90) | 3 |
| Cetaryl isononanoate | 16 |
| Phenoxyethanol | 0,6 |
| Farbpigmente (CI 77891, CI 77492, CI 77491, CI 77499, CI 77007) | 7 |
| EDTA | 1 |
| Glycerin | 6 |
| Natriumchlorid | 2 |
| Wasser 100% | ad 100 |

| Beispielrezeptur 8 | Gew.-% |
|---|---|
| Cetyl Diglyceryl Tris(Trimethylsiloxy)silylethyldimethicone | 3 |
| Cetaryl isononanoate | 16 |
| Phenoxyethanol | 0,6 |
| Farbpigmente (CI 77891, CI 77492, CI 77491, CI 77499, CI 77007) | 7 |
| EDTA | 1 |
| Glycerin | 6 |
| Natriumchlorid | 2 |
| Wasser 100% | ad 100 |

| Beispielrezeptur 9 | Gew.-% |
|---|---|
| Cetyl Diglyceryl Tris(Trimethylsiloxy)silylethyldimethicone | 3 |
| Propylencarbonat | 0,24 |

(fortgesetzt)

| Beispielrezeptur 9 | Gew.-% |
|---|---|
| Cetaryl isononanoate | 12 |
| Octocrylen | 3 |
| Dimethicone / Dimethicone-Crosspolymer | 10 |
| Disteardimonium Hectorit | 1 |
| Farbpigmente (Cl 77891, Cl 77492, Cl 77491, Cl 77499, Cl 77007) | 5 |
| $TiO_2$ | 1 |
| Trisodium EDTA | 1 |
| Propylenglycol | 2 |
| Glycerin | 5 |
| Natriumchlorid | 2 |
| Phenoxyethanol | 0,4 |
| Parfum | 0,2 |
| Butylmethoxydibenzoylmethan | 0,9 |
| Wasser 100% | ad 100 |

| Beispielrezeptur 10 | Gew.-% |
|---|---|
| Cetyl Diglyceryl Tris(Trimethylsiloxy)silylethyldimethicone | 2 |
| Isohexadecane | 3 |
| Dicaprylyl Ether | 5 |
| Isopropylpalmitate | 3 |
| C12-15 Alkylbenzoate | 3 |
| Ethylhexylcocoate | 3 |
| Methylparaben | 0,2 |
| Alpha-Glucosylrutin | 0,1 |
| Trisodium EDTA | 1 |
| Ubichinon | 0,1 |
| Glycerin | 7 |
| Ascorbylphosphat | 0,3 |
| Natriumchlorid | 2 |
| Phenoxyethanol | 0,4 |
| Parfum | 0,2 |
| Wasser 100% | ad 100 |

| Beispielrezeptur 11 | Gew.-% |
|---|---|
| Cetyl Diglyceryl Tris(Trimethylsiloxy)silylethyldimethicone | 0,9 |
| Ethylhexylcocoate | 4 |
| Dicaprylyl Ether | 2 |

(fortgesetzt)

| Beispielrezeptur 11 | Gew.-% |
|---|---|
| Isopropylpalmitate | 2 |
| Caprylic Capric Triglycerid | 3 |
| Farbpigmente (CI 77891, CI 77492, CI 77491, CI 77499, CI 77007) | 5 |
| Trisodium EDTA | 1 |
| Pentylenglycol | 0,3 |
| Glycerin | 5 |
| Natriumchlorid | 1 |
| Phenoxyethanol | 0,4 |
| Parfum | 0,2 |
| Wasser 100% | ad 100 |

| Beispielrezeptur 12 | Gew.-% |
|---|---|
| Cetyl Diglyceryl Tris(Trimethylsiloxy)silylethyldimethicone | 4 |
| Ethylhexylstearate | 5 |
| Dicaprylyl Ether | 5 |
| Diethylhexylbutamidotriazone | 1 |
| Octyldodecanol | 3 |
| Caprylic/Capric Triglyceride | 2 |
| Diethylamino hydroxybenzoyl hexylbenzoate | 0,9 |
| Octylsalicylat | 2 |
| Tocopherylacetat | 0,5 |
| Retinylpalmitat | 0,1 |
| Trisodium EDTA | 1 |
| Methylpropandiol | 2 |
| Octandiol | 0,1 |
| Panthenol | 0,5 |
| Glycerin | 6 |
| Phenoxyethanol | 0,4 |
| Parfum | 0,2 |
| Wasser 100% | ad 100 |

**Patentansprüche**

1. Kosmetische oder dermatologische Wasser-in-Öl- Emulsionen, enthaltend

A) 0,1 bis 10,0 Gewichts-%, bevorzugt 0,2 bis 5,0 Gew.-%, besonders bevorzugt 0,5 bis 4,0 Gew.-%, bezogen auf das Gesamtgewicht der Zubereitung, an Cetyl Diglyceryl Tris-(Trimethylsiloxy)silylethyldimethicone als Emulgator
B) eine polare Ölphase mit maximal 0,95 Gewichts-%, bevorzugt höchstens 0,5 Gewichts-%, besonders bevorzugt höchstens 0,1 Gewichts-% an einem oder mehreren bei Raumtemperatur flüssigen Silikonölen enthalten und ganz besonders bevorzugt gänzlich frei von Silikonölen sind,
C) eine wässrige Phase.

2. Wasser-in-Öl Emulsionen nach einem der vorstehenden Ansprüche, **dadurch gekennzeichnet, dass** die Öle der polaren Ölphase bevorzugt gewählt werden aus der Gruppe der bei Raumtemperatur flüssigen Monoester, Ether, Triglyceride, Alkohole sowie verzweigtkettigen und linearen Kohlenwasserstoffe.

3. Emulsionen nach einem der vorstehenden Ansprüche, **dadurch gekennzeichnet, dass** ihre Viskositäten im Intervall von 100 - 20000 mPa.s, vorzugsweise von 200 - 10000 mPa.s, insbesondere bevorzugt von 300 - 5000 mPa.s bei einer Temperatur von 25 °C und einer Scherrate von ca. 10 s-1 liegen. (Gemessen mit dem Rheomat R 123).

**Claims**

1. Cosmetic or dermatological water-in-oil emulsions comprising

A) 0.1 to 10.0% by weight, preferably 0.2 to 5.0% by weight, particularly preferably 0.5 to 4.0% by weight, based on the total weight of the preparation, of cetyl diglyceryl tris(trimethylsiloxy)silylethyl dimethicone as emulsifier
B) a polar oil phase comprising at most 0.95% by weight, preferably at most 0.5% by weight, particularly preferably at most 0.1% by weight of one or more silicone oils liquid at room temperature, and especially preferably are completely free of silicone oils,
C) an aqueous phase.

2. Water-in-oil emulsions according to any of the preceding claims, **characterized in that** the oils of the polar oil phase are preferably selected from the group of monoesters, ethers, triglycerides, alcohols and branched and linear hydrocarbons that are liquid at room temperature.

3. Emulsions according to either of the preceding claims, **characterized in that** viscosities thereof are in the interval from 100-20 000 mPa.s, preferably from 200 -10 000 mPa.s, particularly preferably from 300 - 5000 mPa.s at a temperature of 25°C and a shear rate of ca. 10 s$^{-1}$ (measured with a Rheomat R 123).

**Revendications**

1. Émulsions eau-dans-huile cosmétiques ou dermatologiques, contenant

A) 0,1 à 10,0 % en poids, préférablement 0,2 à 5,0 % en poids, particulièrement préférablement 0,5 à 4,0 % en poids, par rapport au poids total de la préparation, de cétyle diglycéryle tris-(triméthylsiloxy)silyléthylediméthicone en tant qu'émulsifiant
B) une phase huileuse polaire contenant au maximum 0,95 % en poids, préférablement au plus 0,5 % en poids, particulièrement préférablement au plus 0,1 % en poids d'une ou plusieurs huiles de silicone liquides à température ambiante et tout particulièrement préférablement étant totalement exemptes d'huiles de silicone,
C) une phase aqueuse.

2. Émulsions eau-dans-huile selon l'une quelconque des revendications précédentes, **caractérisées en ce que** les huiles de la phase huileuse polaire sont préférablement choisies dans le groupe des monoesters, des éthers, des triglycérides, des alcools ainsi que des hydrocarbures linéaires et à chaîne ramifiée, liquides à température ambiante.

3. Émulsions selon l'une quelconque des revendications précédentes, **caractérisées en ce que** leurs viscosités se situent dans l'intervalle de 100 à 20 000 mPa.s, de préférence de 200 à 10 000 mPa.s, en particulier préférablement de 300 à 5 000 mPa.s à une température de 25 °C et une vitesse de cisaillement d'environ 10 s$^{-1}$ (mesurées avec le Rhéomat R 123).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **CINDY DELVALLÉ et al.** New Formulation Possibilities with a Water-in-oil Silicone Emulsifier Suitable for PEG-free Systems. *Dow Corning,* 31. Dezember 2014